# EUROPEAN PATENT APPLICATION

(11) **EP 4 678 162 A1**
(43) Date of publication of application: **14.01.2026**
(21) Application number: 24218841.5
(22) Date of filing: 10.12.2024
(51) Int. Cl.: A61K 8/99, A61K 35/747, A61Q 11/00, A61P 31/04, A23L 33/135, A61K 8/00, C12N 1/20, A61P 43/00

(54) **COMPOSITION FOR AMELIORATING, PREVENTING OR TREATING ORAL DISEASES CONTAINING STRAIN COMBINATION OF LACTOBACILLUS GASSERI HHUMIN D AND LACTICASEIBACILLUS PARACASEI OK**

(30) Priority: 11.07.2024 KR 20240091618
(71) Applicant: Bifido Co., Ltd., Gangwon-do 25117 (KR)
(72) Inventor: Park, Myeong Soo, 06091 Seoul (KR); Heo, Keon, 16507 Gyeonggi-do (KR); Moon, Jin Seok, 28221 Chungcheongbuk-do (KR); Yuk, Kyung Jin, 12913 Gyeonggi-do (KR); Lee, Ye Rim, 12913 Gyeonggi-do (KR); Choi, Ji Hye, 13005 Gyeonggi-do (KR); Sim, Seong Rok, 12913 Gyeonggi-do (KR)
(74) Representative: RGTH

(57) **Abstract**

Disclosed is a composition for ameliorating, preventing or treating an oral disease containing a strain combination of *Lactobacillus gasseri* HHuMIN D and *Lacticaseibacillus paracasei* OK. The strain combination is capable of inhibiting oral harmful bacteria, thus being highly effective in ameliorating, preventing, and treating oral diseases including bad breath, tooth decay, and periodontal disease.

## Description

### BACKGROUND OF THE INVENTION

### Field of the Invention

The present invention relates to a composition for ameliorating, preventing or treating an oral disease containing a strain combination of *Lactobacillus gasseri* HHuMIN D and *Lacticaseibacillus paracasei* OK.

### Description of the Related Art

Bad breath refers to an unpleasant smell of exhaled breath derived from the mouth. As bad breath has been reported to have a negative impact on social activities and reflect health, the interest of the general public in bad breath has been gradually increasing for several years, and the number of patients visiting the dentist due to bad breath as a chief complaint is gradually increasing. The prevalence of bad breath in adults is 25-50%, about 25% of which corresponds to chronic bad breath that is severe enough to affect social function.

The known causes of bad breath may be divided into oral and extraoral causes, and it is known that the oral cause accounts for 85-90%. Bad breath due to oral causes results from host factors such as teeth or saliva components and food residues decayed by bacteria. The oral causes include decreased salivary secretion, stomatitis, periodontal disease, inappropriate prosthetics, excessive microbial deposition on the tongue, the consumption of irritating foods, smoking, drinking, and other factors that cause bad breath include increased saliva viscosity, decreased hydrogen ion concentration buffering capacity, increased amount and activity of oral microorganisms, and tongue coating attached to the back of the tongue, oral diseases such as untreated dental caries and periodontal disease, and oral cancer. It is known that extraoral causes include systemic factors such as diabetes, liver disease, kidney disease, and gastrointestinal disease, diseases such as upper respiratory tract infections, otolaryngological diseases including sinusitis, respiratory tract lung diseases, digestive diseases, and psychological factors.

Meanwhile, it is known that bad breath may cause daily stress and symptoms such as anger, depression, anxiety, and obsessive-compulsive disorder, and have a serious impact on emotional and social adaptation issues. In fact, it was found that patients with bad breath have greater psychological damage such as obsessive-compulsive disorder, interpersonal sensitivity, anxiety, and hostility than normal people, and studies have reported that patients with bad breath experience psychological withdrawal or social avoidance because others think negatively of themselves, which may also have a negative impact on their self-image and body image.

There are various methods to manage bad breath symptoms. An oral antiseptic is the most commonly used. An oral antiseptic chemically removes volatile sulfur compounds (VSC), which are the cause of bad breath. However, an oral antiseptic is made of a chemical compound, thus having a risk of damaging oral tissue when used for a long period of time. Although many patients suffer from bad breath, there is currently no appropriate method to ameliorate bad breath symptoms, and the smell is merely temporarily shielded or reduced.

Accordingly, as a result of intense effort, the present inventors found a strain combination having excellent selective antibacterial activity while having low risk of human and environmental toxicity in order to prevent various oral diseases including bad breath, and identified excellent ability thereof to inhibit oral disease-causing bacteria and oral disease. Based on this, the present invention has been completed.

### [Prior art literature]

### [Patent literature]

(Patent literature 1) Korean Patent Publication Laid-open No. 10-2014-0106988 (2014.09.04.) discloses *Lactobacillus sakei* CBNU2567 with anti-cariogenic activity isolated from kimchi.
(Patent literature 2) Korean Patent No. 10-1715163 (2017.03.06.) discloses a food composition for removing bad breath containing a kimchi lactic acid bacteria fermentation product of pine needle and pine cone extracts, and a method of preparing the same.

### SUMMARY OF THE INVENTION

Therefore, the present invention has been made in view of the above problems, and it is an object of the present invention to provide use of a strain combination of *Lactobacillus gasseri* HHuMIN D (KCCM 12724P) and *Lacticaseibacillus paracasei* OK (KCCM 13365P) for ameliorating, preventing or treating an oral disease.

In accordance with an aspect of the present invention, the above and other objects can be accomplished by the provision of a pharmaceutical composition for preventing or treating an oral disease containing a *Lactobacillus gasseri* HHuMIN D strain (KCCM 12724P) and a *Lacticaseibacillus paracasei* OK strain (KCCM 13365P).

The pharmaceutical composition may contain a combination of any one selected from live cells and dead cells of a *Lactobacillus gasseri* HHuMIN D strain (KCCM 12724P), and a culture solution thereof, and any one selected from live cells and dead cells of a *Lacticaseibacillus paracasei* OK strain (KCCM 13365P), and a culture solution thereof.

The oral disease may be selected from bad breath, caries and periodontal disease,

The bad breath may be caused by *Fusobacterium nucleatum,* the caries may be caused by *Streptococcus mutans,* and the periodontal disease may be caused by any one selected from the group consisting of *Fusobacterium nucleatum, Porphyromonas gingivalis,* and *Prevotella intermedia.*

In accordance with another aspect of the present invention, there is provided a food composition for ameliorating an oral disease containing a *Lactobacillus gasseri* HHuMIN D strain (KCCM 12724P) and a *Lacticaseibacillus paracasei* OK strain (KCCM 13365P).

The food composition may contain a combination of any one selected from live cells and dead cells of a *Lactobacillus gasseri* HHuMIN D strain (KCCM 12724P), and a culture solution thereof, and any one selected from live cells and dead cells of a *Lacticaseibacillus paracasei* OK strain (KCCM 13365P), and a culture solution thereof.

In accordance with a further aspect of the present invention, there is provided a composition for suppressing bad breath containing a *Lactobacillus gasseri* HHuMIN D strain (KCCM 12724P) and a *Lacticaseibacillus paracasei* OK strain (KCCM 13365P) .

The composition may contain a combination of any one selected from live cells and dead cells of a *Lactobacillus gasseri* HHuMIN D strain (KCCM 12724P), and a culture solution thereof, and any one selected from live cells and dead cells of a *Lacticaseibacillus paracasei* OK strain (KCCM 13365P), and a culture solution thereof.

### BRIEF DESCRIPTION OF THE DRAWINGS

The above and other objects, features and other advantages of the present invention will be more clearly understood from the following detailed description taken in conjunction with the accompanying drawings, in which:
FIG. 1 is the result of a genealogy analysis of the 16S rRNA base sequence of the strain *Lacticaseibacillus paracasei* OK (KCCM 13365P) of the present invention;
FIG. 2 shows the number of oral harmful bacteria attached to oral epithelial cells upon single treatment and combination treatment of *Lactobacillus gasseri* HHuMIN D and *Lacticaseibacillus paracasei* OK strains;
FIG. 3 shows the degree of inhibition of expression of *mgl* genes (causing bad breath) of *P. gingivalis* strains upon single and combination treatment of *Lactobacillus gasseri* HHuMIN D and *Lacticaseibacillus paracasei* OK strains;
FIG. 4 is a schematic diagram illustrating a human test method;
FIG. 5 shows a change in H₂S (PP set) upon administration of the strain combination according to the present invention;
FIG. 6 shows a change in H₂S (FA set) upon administration of the strain combination according to the present invention;
FIG. 7 shows a change in CH₃SH (PP set) upon administration of the strain combination according to the present invention;
FIG. 8 shows a change in CH₃SH (FA set) upon administration of the strain combination according to the present invention;
FIG. 9 shows a change in (CH₃)₂S (PP set) upon administration of the strain combination according to the present invention;
FIG. 10 shows a change in (CH₃)₂S (FA set) upon administration of the strain combination according to the present invention;
FIG. 11 shows a change in H₂S+CH₃SH+(CH₃)₂S (PP set) upon administration of the strain combination according to the present invention;
FIG. 12 shows a change in H₂S+CH₃SH+(CH₃)₂S (FA set) upon administration of the strain combination according to the present invention;
FIG. 13 shows a change in TPI (tongue plaque index) (PP set) upon administration of the strain combination according to the present invention;
FIG. 14 shows a change in TPI (tongue plaque index) (FA set) upon administration of the strain combination according to the present invention;
FIG. 15 shows a change in GI (gingival index) (PP set) upon administration of the strain combination according to the present invention;
FIG. 16 shows a change in GI (gingival index) (FA set) upon administration of the strain combination according to the present invention;
FIG. 17 shows a change in PI (plaque index) (PP set) upon administration of the strain combination according to the present invention;
FIG. 18 shows a change in PI (plaque index) (FA set) upon administration of the strain combination according to the present invention;
FIG. 19 shows a change in PHP (patient hygiene performance) index (PP set) upon administration of the strain combination according to the present invention;
FIG. 20 shows a change in PHP (patient hygiene performance) index (FA set) upon administration of the strain combination according to the present invention;
FIG. 21 shows a change in *P. gingivalis* (PP set) upon administration of the strain combination according to the present invention;
FIG. 22 shows a change in *P. gingivalis* (FA set) upon administration of the strain combination according to the present invention;
FIG. 23 shows a change in *P. intermedia* (PP set) upon administration of the strain combination according to the present invention;
FIG. 24 shows a change in *P. intermedia* (FA set) upon administration of the strain combination according to the present invention;
FIG. 25 shows a change in *F. nucleatum* (PP set) upon administration of the strain combination according to the present invention;
FIG. 26 shows a change in *F. nucleatum* (FA set) upon administration of the strain combination according to the present invention;
FIG. 27 shows a change in *S. mutans* (PP set) upon administration of the strain combination according to the present invention;
FIG. 28 shows a change in *S. mutans* (FA set) upon administration of the strain combination according to the present invention;
FIG. 29 shows the result of sensory evaluation of bad breath (PP set) upon administration of the strain combination according to the present invention; and
FIG. 30 shows the result of sensory evaluation of bad breath (FA set) upon administration of the strain combination according to the present invention.

### DETAILED DESCRIPTION OF THE INVENTION

In order to avoid confusion due to overlapping content, overlapping content is omitted in the following description. In other words, the content of the present invention is not limited to the content given below and the content of the present invention should be interpreted based on the overall content.

In one embodiment of the present invention, a human test conducted on people with bad breath shows that a combination of a *Lactobacillus gasseri* HHuMIN D strain (KCCM 12724P) and a *Lacticaseibacillus paracasei* OK strain (KCCM 13365P) has excellent inhibitory activity against oral disease-associated harmful bacteria and has excellent antibacterial activity against bad breath-causing harmful bacteria, bad breath alleviation effect, and stability.

The *Lactobacillus gasseri* HHuMIN D strain (KCCM 12724P) according to the present invention is a strain disclosed in Korean Patent No. 10-2198553 (December 29, 2020).

The *Lacticaseibacillus paracasei* OK strain according to the present invention is a lactic acid bacteria derived from human feces, which is deposited with the Korea Center for Microorganism Conservation (KCCM) and was assigned the accession number KCCM 13365P on June 30, 2023.

Accordingly, in one aspect, the present invention is directed to a pharmaceutical composition for preventing or treating an oral disease containing a *Lactobacillus gasseri* HHuMIN D strain (KCCM 12724P) and a *Lacticaseibacillus paracasei* OK strain (KCCM 13365P).

The composition preferably contains a combination of any one selected from live cells and dead cells of a *Lactobacillus gasseri* HHuMIN D strain (KCCM 12724P), and a culture solution thereof, and any one selected from live cells and dead cells of a *Lacticaseibacillus paracasei* OK strain (KCCM 13365P), and a culture solution thereof.

In the composition according to the present invention, the oral disease may be, for example, any one selected from bad breath, caries and periodontal disease.

The bad breath is preferably caused by *Fusobacterium nucleatum,* the caries is preferably caused by *Streptococcus mutans,* and the periodontal disease is preferably caused by any one selected from the group consisting of *Fusobacterium nucleatum, Porphyromonas gingivalis,* and *Prevotella intermedia.*

In one embodiment of the present invention, it was found that the strain combination according to the present invention is highly effective in inhibiting the growth of the oral harmful bacteria exemplified above and in inhibiting the attachment of these oral harmful bacteria to oral epithelial cells. In addition, the excellent bad breath amelioration effect of the strain combination according to the present invention was confirmed by a human test.

*Fusobacterium nucleatum* is a major anaerobic bacterium that produces hydrogen sulfide or methyl mercaptan and is a gram-negative bacterium that normally exists in the oral cavity, causes periodontitis, and produces volatile sulfur compounds to cause bad breath.

*Streptococcus mutans* is a mesophilic bacterium that grows at 18 to 40°C and is a bacterium that ferments sugars in the mouth to produce an acid. *Streptococcus mutans* lives not only in the oral cavity but also in the pharynx and intestines. *Streptococcus mutans* plays an important role in the formation of caries due to the ability thereof to attach to enamel or produce acidic byproducts. *Streptococcus mutans* decomposes sugars to obtain energy, which in turn produces lactic acid, creating an acidic environment, which removes salt from the tooth surface and causes tooth decay by dissolving calcium based thereon.

*Porphyromonas gingivalis* is a part of the main oral microbiota of humans, which is a gram-negative bacterium and an obligate anaerobe. *Porphyromonas gingivalis* lives in periodontal pockets or grooves between teeth and gums, and is mainly found in the oral cavity and is known to be related to periodontal disease, but is also found in the upper gastrointestinal tract, respiratory tract, large intestine, or the like, and is also related to bacterial vaginosis in women. In humans, *Porphyromonas gingivalis* is most associated with the subgingiva of the oral cavity and forms a part of the biofilm that becomes dental plaque. *Porphyromonas gingivalis* causes painful inflammation in periodontal disease. It is known that 10-15% of adults all over the world suffer from such an oral disease.

*Prevotella intermedia* is a gram-negative obligate anaerobic pathogenic bacterium involved in periodontal infections including gingivitis and periodontitis, and is often found in acute necrotizing ulcerative gingivitis. It is known as a major causative agent of periodontal disease, and continues its life while producing various enzymes including proteolytic enzymes to decompose surrounding organic matter. When the organic matter is collagen that forms the gums, periodontal disease occurs.

As used herein, the term "prevention" refers to any action that suppresses or delays the onset of an oral disease affected by administration of the pharmaceutical composition according to the present invention.

As used herein, the term "treatment" refers to any action that ameliorates or beneficially changes symptoms of an oral disease by administration of the pharmaceutical composition according to the present invention.

The strain according to the present invention may contain one or more active ingredients that exhibit the same or similar functions in addition to the ingredient.

The pharmaceutical composition according to the present invention may further contain a pharmaceutically acceptable carrier in addition to the strain according to the present invention.

The type of carrier that may be used in the present invention is not particularly limited and any carrier commonly used in the relevant technical field may be used. Non-limiting examples of the carrier include lactose, dextrose, sucrose, sorbitol, mannitol, saline solution, sterile water, Ringer's solution, buffered saline, albumin injection solution, xylitol, erythritol, maltitol, maltodextrin, glycerol, ethanol, and the like. These may be used alone or in combination of two or more.

In addition, the pharmaceutical composition according to the present invention may further contain other pharmaceutically acceptable additives such as antioxidants, excipients, diluents, buffers or bacteriostatic agents, and the pharmaceutical composition may further contain surfactants, binders, fillers, bulking agents, humectants, disintegrants, dispersants or lubricants, if necessary.

The strain according to the present invention may be contained in the pharmaceutical composition according to the present invention in an amount of 0.00001 wt% to 99.99 wt%, preferably 0.1 wt% to 90 wt%, more preferably 0.1 wt% to 70 wt%, and even more preferably 0.1 wt% to 50 wt%, based on the total weight of the pharmaceutical composition, but is not limited thereto and may be changed depending on the condition of the administration subject, the type of specific disease, the degree of progression, or the like. If necessary, the content of strain may be the same as the total content of the pharmaceutical composition.

That is, the pharmaceutically effective amount, namely, the effective dosage of the pharmaceutical composition according to the present invention may vary depending on the method of formulating the pharmaceutical composition, the method of administration, the time of administration, and/or the route of administration, and may vary depending on various factors including the type and degree of the response to be achieved by administration of the pharmaceutical composition, the type, age, weight, general health condition, symptoms or degree of the disease, gender, diet, and excretion of the subject, drugs used simultaneously or simultaneously in the subject, other components of the composition, and similar factors well known in the medical field, and those having ordinary knowledge in the technical field can easily determine and prescribe an effective dosage for the intended treatment. For example, the daily dosage of the pharmaceutical composition according to the present invention is about 0.01 to 1,000 mg/kg, preferably 0.1 to 100 mg/kg, and may be administered once or in several divided doses a day.

The pharmaceutical composition according to the present invention may be administered once a day or may be administered in several divided doses. The pharmaceutical composition according to the present invention may be administered as an individual therapeutic agent or in combination with other therapeutic agents, and may be administered sequentially or simultaneously with conventional therapeutic agents. Taking all of the factors into consideration, the pharmaceutical composition may be administered in a minimum amount that can obtain the maximum effect without causing side effects, which can be easily determined by those skilled in the art.

The pharmaceutical composition according to the present invention may be further used in combination with various methods such as hormone therapy and drug therapy for preventing or treating the disease.

As used herein, the term "administration" means introducing the pharmaceutical composition according to the present invention to a patient by any appropriate method, each of the administration route and administration method of the pharmaceutical composition according to the present invention may be independent, and any administration route and administration method may be used without specific limitation as long as the pharmaceutical composition can reach the target site.

The pharmaceutical composition may be administered by oral administration or parenteral administration, and may be prepared as various formulations suitable for oral administration or parenteral administration. Preferably, the pharmaceutical composition may be prepared into at least one formulation selected from a toothpaste, a mouthwash, an oral spray, and an oral ointment, but is not limited thereto.

Non-limiting examples of oral administration preparations using the pharmaceutical composition according to the present invention include oily suspensions, troches, lozenges, tablets, water-soluble suspensions, prepared powders, granules, emulsions, hard capsules, soft capsules, syrups, elixirs and the like.

In order to prepare a formulation for oral administration of the pharmaceutical composition of the present invention, a binder such as sorbitol, mannitol, starch, amylopectin, cellulose, lactose, saccharose or gelatin, a lubricant such as magnesium stearate, calcium stearate, sodium stearyl fumarate or polyethylene glycol wax, an excipient such as dicalcium phosphate, a disintegrant such as corn starch or sweet potato starch, a fragrance, syrup, sweetener or the like may be used. Furthermore, in the case of capsules, in addition to the above-mentioned substances, a liquid carrier such as fatty oil may be further used.

Parenteral administration of the pharmaceutical composition of the present invention may be carried out by intramuscular, transdermal or subcutaneous administration, and the composition may be applied or sprayed to a diseased site, but parenteral administration is not limited thereto.

Non-limiting examples of parenteral preparations using the pharmaceutical composition of the present invention include injections, ointments, powders for application, oils, aerosols for sprays, creams, and the like.

In order to prepare a formulation for parenteral administration of the pharmaceutical composition of the present invention, sterile aqueous solutions, non-aqueous solvents, suspensions, emulsions, lyophilizates, external preparations, and the like may be used. The non-aqueous solvents and suspensions may include vegetable oils such as olive oil, injectable esters such as propylene glycol, polyethylene glycol, and ethyl oleate and the like.

When the pharmaceutical composition of the present invention is formulated as an injection solution, it is obtained by mixing the pharmaceutical composition with a stabilizer or buffer in the presence of water to prepare a solution or suspension, and preparing the solution or suspension as a unit formulation such as an ampoule or vial.

When the pharmaceutical composition of the present invention is formulated as an aerosol, a propellant or the like may be blended with an additive so as to disperse the water-dispersed concentrate or wet powder.

When the pharmaceutical composition of the present invention is formulated into an ointment, oil, cream, powder for application, external skin preparation, or the like, the formulation may be prepared using animal oil, vegetable oil, wax, paraffin, polyethylene glycol, silicone, bentonite, silica, talc, starch, tragacanth, cellulose derivatives, zinc oxide, or the like as a carrier.

Accordingly, in another aspect, the present invention is directed to a food composition for ameliorating an oral disease containing a *Lactobacillus gasseri* HHuMIN D strain (KCCM 12724P) and a *Lacticaseibacillus paracasei* OK strain (KCCM 13365P).

The composition preferably contains a combination of any one selected from live cells and dead cells of a *Lactobacillus gasseri* HHuMIN D strain (KCCM 12724P), and a culture solution thereof, and any one selected from live cells and dead cells of a *Lacticaseibacillus paracasei* OK strain (KCCM 13365P), and a culture solution thereof.

In one embodiment of the present invention, it was found that the strain combination according to the present invention is highly effective in inhibiting the growth of the oral harmful bacteria exemplified above and in inhibiting the attachment of these oral harmful bacteria to oral epithelial cells. In addition, the excellent bad breath amelioration effect of the strain combination according to the present invention was confirmed by a human test.

The content of the strain according to the present invention in the food composition is not particularly limited and may be changed depending on the condition of the administration subject, the type of specific disease, the degree of progression, or the like. If necessary, the content of the strain may be the same as the total content of the food composition.

For example, the food composition according to the present invention may be prepared as a formulation selected from the group consisting of noodles, gum, dairy products, ice cream, meat, grains, caffeinated beverages, general beverages, chocolate, bread, snacks, confectionery, candy, pizza, jelly, sauce, alcoholic beverages, alcohol, vitamin complexes, and other health supplements. Preferably, the food composition according to the present invention is provided as at least one formulation selected from gum, candy and tablet, but is not limited thereto.

When the food composition according to the present invention is used as a food additive, it may be added as it is or used in combination with other foods or food ingredients, and may be used appropriately in accordance with a conventional method.

In addition, the food composition includes a meaning of a health functional food. As used herein, the term "health functional food" refers to a food prepared from or treated with raw materials or ingredients having functionality useful for the human body according to Act No. 6727 on Health Functional Foods, and the term "functional" means intake of food with the goal of obtaining beneficial effects for health such as regulation of nutrients appropriate for structures and functions of the human body or physiological effects.

In another aspect, the present invention is directed to a composition for suppressing bad breath containing a *Lactobacillus gasseri* HHuMIN D strain (KCCM 12724P) and a *Lacticaseibacillus paracasei* OK strain (KCCM 13365P).

The composition preferably contains a combination of any one selected from live cells and dead cells of a *Lactobacillus gasseri* HHuMIN D strain (KCCM 12724P), and a culture solution thereof, and any one selected from live cells and dead cells of a *Lacticaseibacillus paracasei* OK strain (KCCM 13365P), and a culture solution thereof.

In the present invention, the composition for suppressing bad breath is preferably formulated as a toothpaste, a mouth rinse, or a mouthwash, and any desirable formulation may be used depending on the method of use without limitation.

For example, when the oral composition is formulated as a toothpaste, it may contain an abrasive, a humectant, a foaming agent, a thickener, a preservative, a sweetener, a flavoring agent, or other additives.

The abrasive is an ingredient to remove dental plaque, tartar, or the like attached to teeth, and may include colloidal silicon dioxide, calcium dihydrogen phosphate, calcium carbonate, aluminum hydroxide, precipitated silica, hydrous silicic acid, or the like.

The humectant is an ingredient to suppress evaporation of water in the toothpaste composition and prevent the composition from solidifying when exposed to air, and may include at least one polyhydric alcohol selected from glycerin, sorbitol solution, non-crystallized sorbitol solution, polyethylene glycol, and propylene glycol.

The foaming agent is an ingredient to emulsify the water-soluble ingredients and oil-soluble ingredients in the toothpaste composition and has a cleansing effect in the oral cavity, and may be an anionic or nonionic surfactant selected from sodium lauryl sulfate, sodium N-lauroyl sarcosinate, N-long-chain acyl glutamate, sucrose fatty acid ester, polyoxyethylene hydrogenated castor oil, sorbitan fatty acid ester, a polyoxyethylene polyoxypropylene copolymer, or a combination thereof.

The thickener is an ingredient that binds a liquid ingredient to an inorganic powder ingredient insoluble in water in the toothpaste composition to prevent phase separation over time, and imparts viscosity to the composition, and may include sodium carboxymethyl cellulose, carrageenan, xanthan gum, polyvinyl pyrrolidone, sodium alginate, or other gum.

The preservative is an ingredient that extends a preservation period of the toothpaste composition and may include parahydroxybenzoate ester, benzoic acid, sodium benzoate, or the like.

The sweetener and the flavoring agent are ingredients that improve the taste when brushing teeth and provide a refreshing aftertaste after brushing teeth. The sweetener may include sodium saccharin, aspartame, stevioside, licorice acid, or the like, and the flavoring agent may include a mixture of peppermint, spearmint oil, menthol, carvone, anethole, eugenol, or the like.

The other additives may include a whitening agent such as titanium dioxide, and an edible coloring agent such as Blue No. 1 as a coloring agent.

For example, when the oral composition is formulated as a mouthwash, it may contain a humectant, a sweetener, a foaming agent, a solubilizing agent, an astringent, a preservative, a coloring agent, a flavoring agent, a solvent, or the like.

The ingredients of the mouthwash may be the same as the ingredients of the toothpaste composition, except that zinc chloride may be used as an astringent, and ethanol and purified water may be used as solvents.

For example, when the oral composition is formulated as a mouthwash, it may contain a foaming agent, a dehumidifying agent, a foaming agent, a binder, a lubricant, a flavoring agent, a solvent, or the like.

The foaming agent is an ingredient for oral washing, and may include at least one selected from the group consisting of sodium bicarbonate, ammonium bicarbonate, potassium bicarbonate, and iron carbonate.

The dehumidifying agent is an ingredient to formulate the mouthwash into an anhydrous tablet, and may be sodium carbonate.

The binder may be a water-soluble polymer, preferably carboxyl cellulose or polyvinyl pyrrolidone.

The lubricant may be selected from the group consisting of magnesium stearate, stearic acid, stearyl alcohol, sucrose fatty acid ester, talc, light anhydrous silicic acid, and sodium benzoate, and is preferably sodium benzoate.

Hereinafter, the present invention will be described in more detail with reference to the following examples or experimental examples. However, these examples or experimental examples are provided only for illustration of the present invention and should not be construed as limiting the scope of the present invention.

### [Example 1: Identification of Lacticaseibacillus paracasei OK strain]

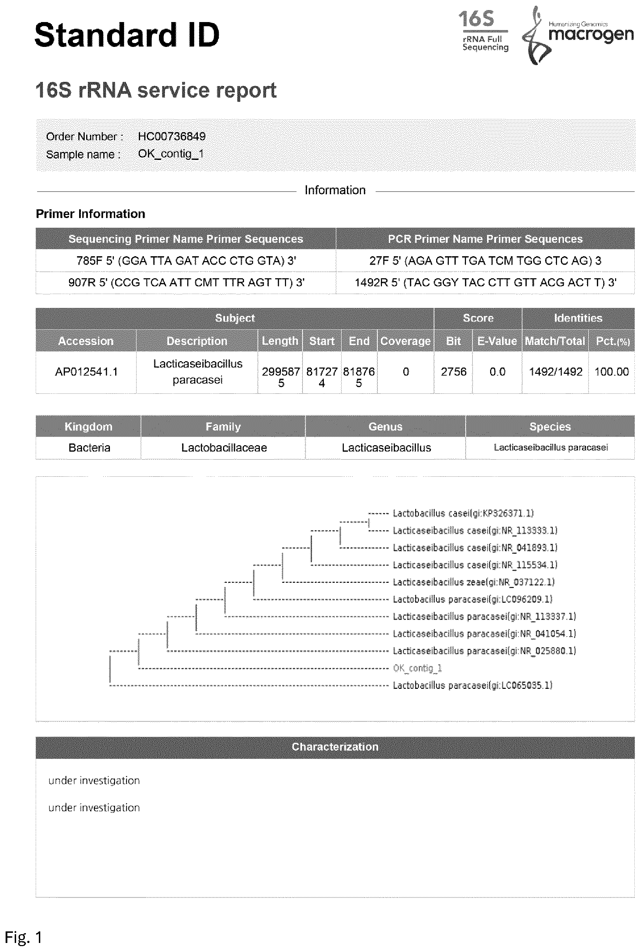

In order to genetically identify *Lacticaseibacillus paracasei* OK (hereinafter referred to as "LP OK"), which is a strain derived from human feces, 16S rRNA sequencing was performed and molecular phylogenetic status was also determined based on the base sequence (FIG. 1). FIG. 1 shows the results of genealogy analysis for the 16S rRNA base sequence of *Lacticaseibacillus paracasei* OK (KCCM 13365P), which is a strain of the present invention.

The 16S rRNA base sequence of the "LP OK" strain was multiple-aligned with the 16S rRNA sequence of *Lacticaseibacillus sp.* listed in GeneBank to obtain a similarity matrix and obtain a genealogy. As a result, the range of 16S rRNA homology was found to be 100%.

That is, the "LP OK" strain had a high homology of 100% with *Lacticaseibacillus paracasei.*

Accordingly, the *Lacticaseibacillus paracasei* OK strain of the present invention was deposited with the Korean Culture Center of Microorganisms (KCCM) on June 30, 2023 under the accession number KCCM 13365P.

### [Example 2: Evaluation of oral disease amelioration efficacy of L. gasseri HHuMIN D and L. Paracasei OK strain combination]

### 1. Evaluation of oral pathogenic bacteria adhesion inhibition efficacy in oral epithelial cells

Each of *L. gasseri* HHuMIN D strain and *L. paracasei* OK strain was cultured in an optimal growth medium. Then, the pellet obtained by centrifugation at 4,000 rpm for 10 minutes was diluted with RPMI 1640 to a concentration of 1.0 × 108 CFU/mL to prepare a dilution.

The *L. gasseri* HHuMIN D strain dilution and *L. paracasei* OK strain dilution were mixed in equal volumes to prepare a strain composition.

Meanwhile, KB cells (human mouth epithelial cells; Korean Cell Line Bank 10017) were cultured in RPMI 1640 with 300 mg/L L-glutamine, 10% fetal bovine serum, 100 µg/mL streptomycin, and 100 U/mf penicillin under 5% CO₂ at 37°C.

Then, KB cells were seeded at 3.0 × 10⁵ cells/well in a 24-well plate (SPL Life Sciences Co., Ltd. Pocheon, Korea) and cultured under 5% CO₂ at 37°C until a cell monolayer was formed. Then, *L. gasseri* HHuMIN D, *L. paracasei* OK, and oral pathogen cultured solutions were centrifuged at 4,000 rpm for 10 minutes and the resulting pellets were diluted to a concentration of 1.0 × 10⁸ CFU/mL with RPMI 1640. For the attachment inhibition test, equal amounts of *L. gasseri* HHuMIN D dilution or/and LP OK strain dilution and the oral pathogen dilutions (1.0 × 10⁸ CFU/mL) were mixed and 2 mℓ of the resulting mixture was added to the cell monolayer from which the supernatant from each well had been removed.

For attachment, the plate was incubated under 5% CO₂ at 37°C for 1 hour. Then, the supernatant from each well was removed, washed twice with PBS, and then treated with 200 µℓ trypsin-EDTA (Welgene, Daegu, Korea) for 5 minutes to collect the cells and attached bacteria. The number of bacteria attached to each KB cell was calculated using the real-time PCR method.

Specifically, a MagMax microbiome ultra nucleic acid isolation kit (Thermo Fisher Scientific, St. Peters, MO, USA) was used to extract the genomic DNA of the attached bacteria from the harvested pellet. In order to measure the number of bacteria, 1.0 X 10⁸ CFU of bacterial genomic DNA was extracted using the same method and diluted using serial dilution to create a standard curve.

Meanwhile, the experiment was conducted using the same method as above, except that 2 mℓ of the oral pathogen dilution (1.0 × 10⁸ CFU/mL) was added to the KB cell monolayer as a positive control group. The adhesion inhibition effect was evaluated based on the ratio of the experimental group to the positive control group, the results of the single strain treatment are shown in Table 1 below, and the results of treatment with the strain combination are shown in Table 2 below.

**[Table 1]**

| **Strains** | **Experimental Groups** | **Number of bacteria (Total cells)** | **Inhibitory rate (%)** |
|---|---|---|---|
| *S. mutans* | *S. mutans* KCTC 3065 | 799563 ± 95307 | |
| | *S. mutans* KCTC 3065+*L*. *paracasei* OK | 551793 ± 21895 | 30.99 ± 2.74 |
| | *S. mutans* KCTC 3065+*L*. *gasseri* HHuMIN D | 745153 ± 62340 | 6.81 ± 7.80 |
| *F*. *nucleatum* | *F. nucleatum* KCTC 2640 | 619282 ± 147762 | |
| | *F. nucleatum* KCTC 2640+*L*. *paracasei* OK | 515447 ± 102127 | 16.77 ± 16.49 |
| | *F. nucleatum* KCTC 2640+*L*. *gasseri* HHuMIN D | 322642 ± 60583 | 47.90 ± 9.78 |
| *P*. *gingivali* s | *P. gingivalis* KCTC 5352 | 1035773 ± 144433 | |
| | *P. gingivalis* KCTC 5352+*L*. *paracasei* OK | 795904 ± 43994 | 23.16 ± 4.25 |
| | *P. gingivalis* KCTC 5352+*L*. *gasseri* HHuMIN D | 222903 ± 9557 ** | 78.48 ± 0.92 |
| *P*. *intermedi a* | *P. intermedia* KCTC 15693 | 3601149 ± 265550 | |
| | *P. intermedia* KCTC 15693+*L*. *paracasei* OK | 423306 ± 1446 *** | 88.25 ± 0.04 |
| | *P. intermedia* KCTC 15693+*L*. *gasseri* HHuMIN D | 2778351 ± 250457 | 22.85 ± 6.95 |
| | | | |

Expressed as mean ± standard error. Statistical significance: *p<0.05, **p<0.01, ***p<0.001.

**[Table 2]**

| **Strains** | **Experimental Groups** | **Number of bacteria (Total cells)** | **Inhibitory rate (%)** |
|---|---|---|---|
| *S*. *mutans* | *S. mutans* KCTC 3065 | 897246 ± 28537 | |
| | *S. mutans* KCTC 3065+*L*. *paracasei OK+L. gasseri* HHuMIN D | 319438 ± 27129 *** | 64.40 ± 3.02 |
| *F*. *nucleatu m* | *F. nucleatum* KCTC 2640 | 154196 ± 8665 | |
| | *F. nucleatum* KCTC 2640+*L*. *paracasei OK+L. gasseri* HHuMIN D | 55453 ± 22790 * | 64.04 ± 14.78 |
| *P*. *gingivalis* | *P. gingivalis* KCTC 5352 | 1035773 ± 144433 | |
| | *P. gingivalis* KCTC 5352+*L*. | 201798 ± 9960 ** | 80.52 ± |
| | *paracasei OK+L. gasseri* HHuMIN D | | 0.96 |
| *P*. *intermed ia* | *P. intermedia* KCTC 15693 | 3601149 ± 265550 | |
| | *P. intermedia* KCTC 15693+*L*. *paracasei OK+L. gasseri* HHuMIN D | 2740438 ± 168972 | 23.90 ± 4.69 |

Expressed as mean ± standard error. Statistical significance: *p<0.05, **p<0.01, ***p<0.001.

The result of the experiment shows that all of the group treated with *L. gasseri* HHuMIN D alone, the group treated with *L. paracasei* OK alone and the group treated with a strain combination thereof exhibited adhesion inhibition efficacy against all oral pathogenic bacteria.

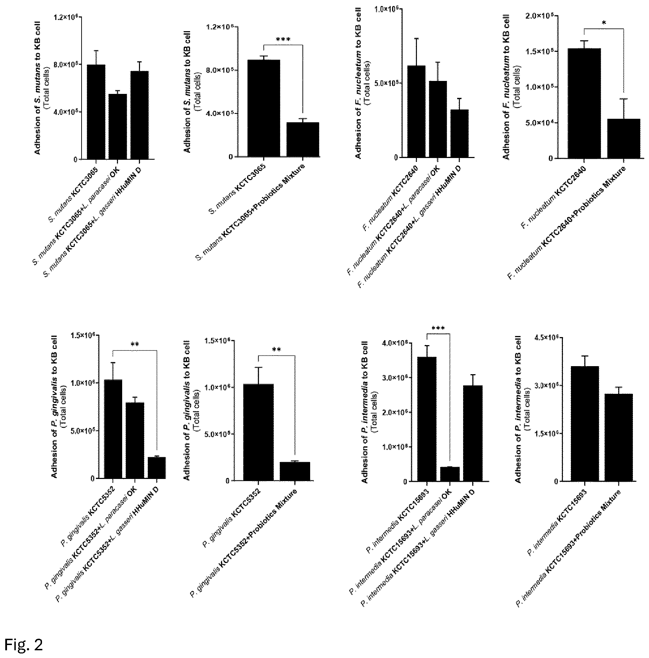

In particular, the group treated with the combination of *L. gasseri* HHuMIN D and *L. paracasei* OK strains exhibited significantly higher adhesion inhibition efficacy against all oral pathogenic bacteria excluding *Privotella intermedia* than the group treated with a single strain, and in particular, exhibited the best adhesion inhibition efficacy against *P*. *gingivalis* (FIG. 2). FIG. 2 has the results of measurement of the number of oral pathogenic bacteria attached to oral epithelial cells upon single or combination treatment of *L*. *gasseri* HHuMIN D and *L. paracasei* OK strains.

### 2. Evaluation of antibacterial activity against oral pathogens

*L. gasseri* HHuMIN D and *L. paracasei* OK were cultured anaerobically at 37°C in MRS borth (BD Difco^{™}) supplemented with 0.05% L-cysteine. *S. mutans* KCTC 3065 was cultured anaerobically in BHI broth (BD Difco^{™}) and *F. nucleatum* KCTC 2640 was cultured anaerobically in RCM (BD Difco^{™}) at 37°C. *P. gingivalis* KCTC 5352 and *P*. intermedia KCTC 15693 were cultured anaerobically in KCOM1 (tryptic soy broth 7.5 g, yeast extract 5 g, 0.025% resazurin, 0.05% hydrogen chloride, 5 µg/mℓ hemin, 2 µg/mℓ vitamin K1) at 37°C.

In order to measure the degree of inhibition of oral pathogenic bacteria growth by a combination of *L. gasseri* HHuMIN D and *L. paracasei* OK strains, *L. gasseri* HHuMIN D, *L*. *paracasei* OK, and four types of oral pathogenic bacteria were co-cultured. When *L. gasseri* HHuMIN D and *L. paracasei* OK combination strains were co-cultured with oral pathogenic bacteria, MRS (0.05% L-cysteine) and the optimal growth medium for each oral pathogenic bacteria were mixed in a volume ratio of 1:1.

The mixed culture solution thus prepared was inoculated with the combination of *L. gasseri* HHuMIN D and *L. paracasei* OK strains and oral pathogenic bacteria at a concentration of 1 × 10⁷ CFU/mL, and then anaerobically cultured at 37°C for 18 to 24 hours. The number of bacteria was measured from the cell pellet obtained by sampling 1 mf of the culture solution. The number of bacteria was calculated by converting the standard curve into the real-time PCR Ct value, and the degree of inhibition of the growth of oral pathogenic bacteria by the treatment with the combination strains of *L. gasseri* HHuMIN D and *L. paracasei* OK is shown in Table 3 below.

**[Table 3]**

| **Strains** | **Experimental Groups** | **Number of bacteria (cells/mℓ)** | **Inhibitory rate (%)** |
|---|---|---|---|
| ***S*. *mutans*** | *S. mutans* KCTC 3065 | 1.4×10¹⁰ ± 1.1×10⁹ | |
| | *S. mutans* KCTC 3065+*L*. *paracasei* OK+ *L. gasseri* HHuMIN D | 2.5×10⁸ ± 1.2×10⁸** | 98.2 |
| ***F*. *nucleatu m*** | *F. nucleatum* KCTC 2640 | 3.5×10⁴ ± 2.4×10⁴ | |
| | *F. nucleatum* KCTC 2640+*L*. *paracasei* OK+ *L. gasseri* HHuMIN D | 3.0×10³ ± 1.1×10³ | 91.2 |
| ***P*. *gingival is*** | *P. gingivalis* KCTC 5352 | 2.4×10⁹ ± 3.3×10⁷ | |
| | *P. gingivalis* KCTC 5352+*L*. *paracasei* OK+ *L. gasseri* HHuMIN D | 2.0×10⁷ ± 4.2×10⁶ *** | 99.1 |
| ***P***. ***intermed ia*** | *P. intermedia* KCTC 15693 | 1.3×10⁹ ± 3.9×10⁷ | |
| | *P. intermedia* KCTC 15693+*L*. *paracasei* OK+ *L. paracasei* OK | 4.2×10⁷ ± 1.3×10⁶ *** | 96.6 |

Expressed as mean ± standard error. Statistical significance: *p<0.05, **p<0.01, ***p<0.001.

The result of the experiment shows that, when the *L*. *gasseri* HHuMIN D and *L. paracasei* OK combination strains were co-cultured with oral pathogenic bacteria, the growth of *S. mutans* was inhibited by 98.2%, the growth of F. nucleatum was inhibited by 91.2%, the growth of *P. gingivalis* was inhibited by 99.1%, and the growth of *P*. *intermedia* was inhibited by 96.6%, compared to the positive control group. Therefore, it was determined that the combination of *L. gasseri* HHuMIN D and *L. paracasei* OK strains had excellent antibacterial activity against oral pathogenic bacteria.

### 3. Evaluation of mgl gene (bad breath inducer) expression inhibition activity

*P. gingivalis* is known to have a mgl gene encoding METase (L-methionine-α-deamino-γ-mercaptomethane-lyase), which produces volatile sulfur compounds from L-methionine and causes bad breath in the mouth.

Here, in order to determine the mgl gene expression inhibition activity of the combination of *L. gasseri* HHuMIN D and *L. paracasei* OK strains, *L. gasseri* HHuMIN D and *L. paracasei* OK were each cultured in MRS broth (0.05% L-cysteine) and the obtained culture supernatants were collected. 1 × 10⁷ CFU of *P. gingivalis* cells were mixed in KCOM1 medium containing 20% of the culture supernatant and anaerobically cultured at 37°C for 18 hours.

The culture supernatants of *L. gasseri* HHuMIN D and *L. paracasei* OK were mixed in a ratio of 1:1 to prepare a mixed supernatant. When the mixed supernatant was treated, *P*. *gingivalis* cells were mixed in KCOM1 medium containing 20% of the mixed supernatant and anaerobically cultured at 37°C for 18 hours. The control group was prepared by replacing 20% of the culture supernatant with a 20% dilution of MRS broth (0.05% L-cysteine) and culturing the same under the same conditions. After culture, the cells were recovered from 1 mℓ of the culture solution, and genomic DNA was extracted therefrom, and then assayed by real-time PCR. The analyzed Ct was analyzed by the 2-^{ΔΔCt} method and the expression level of the *mgl* gene in the experimental group compared to the control group was expressed as a relative fold change.

The result of the experiment shows that the expression level of the *mgl* gene was reduced by 60% when treated with the culture supernatant of *L. gasseri* HHuMIN D and was reduced by 86% when treated with the culture supernatant of *L*. *paracasei* OK.

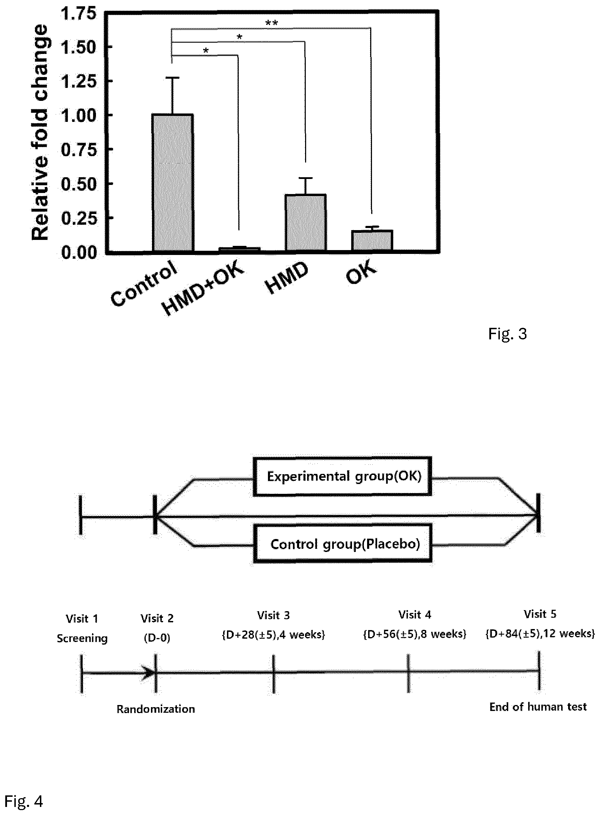

On the other hand, when treated with a 1:1 mixture of the culture supernatants of *L. gasseri* HHuMIN D and *L. paracasei* OK (strain combination), the expression level of the *mgl* gene was reduced by 98%, which indicates that the combination of *L. gasseri* HHuMIN D and *L. paracasei* OK strains had excellent deodorizing activity (FIG. 3). FIG. 3 shows the degree of inhibition of the expression of the *mgl* gene, a cause of bad breath, in the *P. gingivalis* strain upon single strain treatment and strain combination treatment of *L*. *gasseri* HHuMIN D and *L. Paracasei* OK strains.

### [Example 3: Preparation of tablet containing strain combination according to Example 2]

- Main ingredient name: combination of *L. gasseri* HHuMIN D and *L. paracasei* OK
- Appearance and formulation: Tablet containing light yellow powder
- Content: 800 mg/tablet
- Storage method: Refrigerated (2-8 °C) storage
- Usage method and dose: Once a day, one tablet at a time, oral administration after brushing teeth before bedtime (melts in the mouth)
   (1.0 × 10⁹ CFU/day of combination of *L. gasseri* HHuMIN D and *L. paracasei* OK)
- Ingredient name and mixing ratio: Per tablet (800 mg)

**[Table 4]**

| **Name of ingredient** | **Mix rate (%)** | **Content (mg)** |
|---|---|---|
| Composition of *L. gasseri* HHuMIN D and *L. paracasei* OK (Example 2) | 3.600 | 28.800 |
| Isomalt | 47.700 | 381.600 |
| Sorbitol | 47.700 | 381.600 |
| Magnesium stearate | 1.000 | 8.000 |
| Total | 100.00 | 800.000 |

### [Comparative Example 1: Preparation of placebo tablet]

- Main ingredient name: Isomalt and sorbitol
- Appearance and formulation: Tablet containing light yellow powder
- Content: 800 mg/tablet
- Storage method: Refrigerated (2-8 ° C) storage
- Usage method and dose: Once a day, one tablet at a time, oral administration after brushing teeth before bedtime (melts in the mouth)
- Ingredient name and mixing ratio: Per tablet (800 mg)

**[Table 5]**

| **Name of ingredient** | **Mix rate (%)** | **Content (mg)** |
|---|---|---|
| **Isomalt** | 49.396 | 395.168 |
| **Sorbitol** | 49.396 | 395.168 |
| **Magnesium stearate** | 1.000 | 8.000 |
| **Yellow FY201017** | 0.148 | 1.184 |
| **Yellow No. 4 A.L** | 0.006 | 0.048 |
| **Kaoliang color** | 0.054 | 0.432 |
| **Total** | **100.00** | **800.000** |

### [Experimental Example 1: Human Test]

### 1. Human Subject

The human test subjects who voluntarily signed the human test consent form were randomly assigned to either the experimental group or the control group in the order of registration after determining whether they met the selection/exclusion criteria through an on-site evaluation. The number of human subjects assigned to each of the experimental group and the control group was 40. The assigned human test subjects were instructed to administer the tablets according to Example 3 (experimental group) and Comparative Example 1 (control group) once a day (1 tablet) for 12 weeks, and a schematic diagram illustrating the human test method is shown in FIG. 4.

The selection and exclusion criteria for the human subjects are as follows.

### 1) Selection Criteria

A. Adult men and women aged 19 years or older and 70 years or younger
B. Those who have two or more of three specific harmful bacteria (*F. nucleatum, P. gingivalis, P. intermedia*) in the oral microbiome measurement
C. Those whose a total of H₂S, CH₃SH, and (CH₃)₂S in the VSC test in Visit 1 and Visit 2 is 2.0 ng/10 mℓ or higher

### 2) Exclusion criteria

A. Those currently receiving treatment for severe cardiovascular, immune, respiratory, urinary, nervous, musculoskeletal or infectious diseases, or malignant tumors
B. Those who are currently being treated for or have a history of systemic diseases (liver disease, kidney disease, Sjogren's syndrome, and rheumatic disease) that may cause bad breath (however, participation in the test is possible at the investigator's discretion in consideration of the condition of the subject of the human test)
C. Those with severe dental diseases (periodontitis, dental caries, xerostomia, and the like) (those with dental caries of enamel can participate in the test, but those with one or more dentin caries cannot participate in the test)
D. Those currently being treated for sinusitis or rhinitis
E. Those currently being treated for chronic gastritis
F. Current smokers
G. Those who take psychiatric medications such as antidepressants or Parkinson's disease medications within 1 month based on Visit 1
H. Those who took periodontal disease auxiliary treatment within 3 months based on Visit 1
I. Those who received scaling within 3 months based on Visit 1
J. Those who administered antibiotics or antiinflammatory medications within 2 weeks based on Visit 1
K. Those who have been administered or consumed probiotics, prebiotics, or lactic acid bacteria products continuously (more than 4 times a week) within 2 weeks of Visit 1
L. Patients with uncontrolled hypertension (systolic blood pressure of 160 mmHg or higher or diastolic blood pressure of 100 mmHg or higher, based on measurement on subjects of human tests after 10-minutes rest)
M. Patients with uncontrolled diabetes (fasting blood sugar of 180 mg/dL or higher)
N. Those that have AST (GOT) or ALT (GPT) 3 times or higher than the upper limit of normal AST or ALT in the test institution
Aa. Those that have creatinine 2 times or higher than the upper limit of normal creatinine in the testing institution
Ab. Those who are pregnant or lactating or plan to become pregnant during the period of this human test
Ac. Those who are sensitive or allergic to the food ingredients used in this human test
Ad. Those who have participated in another interventional clinical trial (including human application trials) within 1 month (30 days) of Visit 1, or who plan to participate in another interventional clinical trial (including human tests) after the start of this human test.
Ae. Those who the investigator determines are not suitable for this human test.

### 2. Evaluation of efficacy of oral antibacterial improvement and bad breath amelioration

### 1) Primary efficacy variable

The comparison in changes of VSC (volatile sulfur compounds) between the groups before and after intake, as a primary efficacy variable, was analyzed using a paired t-test, and the degree of change between the experimental group and the control group at each time point was evaluated for statistically significant differences by performing a two-sample t-test or Wilcoxon rank sum test depending on whether or not normality was satisfied. ANCOVA was performed using the baseline of the relevant efficacy variable as a covariate, and in addition, when there was a statistically significant difference between the groups in the demographic and lifestyle survey and the difference was clinically significant, the corresponding baseline characteristics were added as covariates.

### 2) Secondary efficacy variables

The comparison between the groups in secondary efficacy variables, tongue plaque index (TPI), gingival index (GI), plaque Index (PI), parent hygiene performance (PHP) index, and oral microbiome measurement changes before and after intake was analyzed using a paired t-test. The degree of change between the experimental group and the control group at each time point was evaluated for statistically significant differences by performing a two-sample t-test or Wilcoxon rank sum test depending on whether normality was satisfied. ANCOVA was performed with the baseline of the relevant validity evaluation variable as a covariate, and in addition, when there was a statistically significant difference between the groups in the demographic and lifestyle survey and the difference was clinically significant, the corresponding baseline characteristics were added as covariates.

### 3) Results of efficacy evaluation

### 1) VSC (volatile sulfur compounds)

Three types of VSC (volatile sulfur compounds), namely, H₂S, CH₃SH, and (CH₃)₂S, were measured using oral chroma at Visits 1, 2, 3, 4, and 5. The measurement was conducted once per visit and the measurements were started after the mouth is closed for 5 minutes. Measurements were conducted in the morning on an empty stomach (8 hours or more) without brushing teeth or drinking water. With the mouth closed, a 1 mℓ syringe to collect oral gas was inserted deep into the mouth of the subject so that the tip of the syringe did not contact tongue or saliva to collect the gas.

### A. Change in H₂S

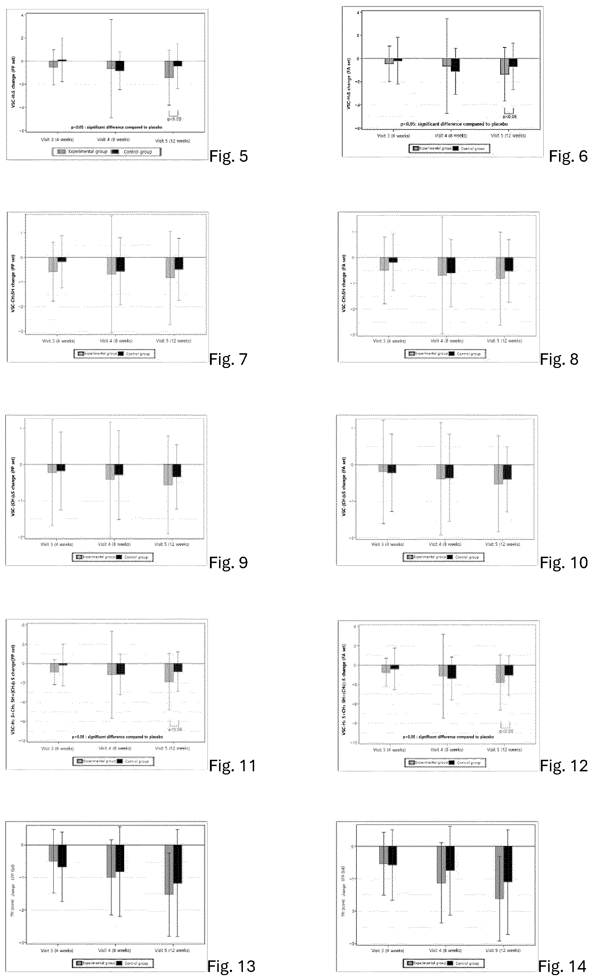

The result of analysis using a PP set (per protocol set) shows that the experimental group exhibited a H₂S decrease of 1.43±2.39 ng/10 mℓ after 12 weeks of intake (p=0.0010), and the control group exhibited a H2S decrease of 0.44±1.95 ng/10 mℓ (p=0.1987) (FIG. 5) . FIG. 5 shows a H₂S change (PP set) upon administration of the strain combination according to the present invention.

The result of analysis using a FA set (full analysis set) shows that, after 12 weeks of intake, the experimental group exhibited a H₂S decrease of 1.36±2.31 ng/10 mℓ (p=0.0007) and the control group exhibited a H₂S decrease of 0.69±2.00 ng/10 mℓ (p=0.0347) (FIG. 6). FIG. 6 shows a H₂S change (FA set) upon administration of the strain combination according to the present invention.

### B. Change in CH₃SH

The result of analysis using a PP set (per protocol set) shows that, after 12 weeks of intake, the experimental group exhibited a CH₃SH decrease of 0.83±1.89 ng/10 mL (p=0.0122), and the control group exhibited a CH₃SH decrease of 0.50±1.26 ng/10 mL (p=0.0281) (FIG. 7). FIG. 7 shows a CH₃SH change (PP set) upon administration of the strain combination according to the present invention.

The result of analysis using a FA set (full analysis set) shows that, after 12 weeks of intake, the experimental group exhibited a CH₃SH decrease of 0.82±1.81 ng/10 mL (p=0.0072) and the control group exhibited a CH₃SH decrease of 0.5211.22 ng/10 mL (p=0.0102) (FIG. 8). FIG. 8 shows a CH₃SH change (FA set) upon administration of the strain combination according to the present invention.

### C. Change in (CH₃)₂S

The result of analysis using a PP set (per protocol set) shows that, after 12 weeks of intake, the experimental group exhibited a (CH₃)₂S decrease of 0.57±1.35 ng/10 mL (p=0.0162), and the control group exhibited a (CH3)2S decrease of 0.35±0.89 ng/10 mL (p=0.0298) (FIG. 9). FIG. 9 shows a (CH₃)₂S change (PP set) upon administration of the strain combination according to the present invention.

The result of analysis using a FA set (full analysis set) shows that, after 12 weeks of intake, the experimental group exhibited a (CH₃)₂S decrease of 0.52±1.31 ng/10 mL (p=0.0168) and the control group exhibited a (CH₃)₂S decrease of 0.40±0.89 ng/10 mL (p=0.0066) (FIG. 10). FIG. 10 shows a (CH₃)₂S change (FA set) upon administration of the strain combination according to the present invention.

### D. Change in H₂S+CH₃SH+(CH₃)₂S

The result of analysis using a PP set (per protocol set) shows that, after 12 weeks of intake, the experimental group exhibited a H₂S+CH₃SH+(CH₃)₂S decrease of 2.83±4.40 ng/10 mL (p=0.0005) and the control group exhibited a H₂S+CH₃SH+(CH₃)₂S decrease of 1.28±3.07 ng/10 mL (p=0.0205) (FIG. 11). FIG. 11 shows a H₂S+CH₃SH+(CH₃)₂S change (PP set) upon administration of the strain combination according to the present invention.

The result of analysis using a FA set (full analysis set) shows that, after 12 weeks of intake, the experimental group exhibited a H₂S+CH₃SH+(CH₃)₂S decrease of 2.71±4.25 ng/10 mL (p=0.0003) and the control group exhibited a H₂S+CH₃SH+(CH₃)₂S decrease of 1.61±3.03 ng/10 mL (p=0.0017) (FIG. 12). FIG. 12 shows a H₂S+CH₃SH+(CH₃)₂S change (FA set) upon administration of the strain combination according to the present invention.

### 2) Change in TPI (tongue plaque index)

TPI (tongue plaque index) was conducted on Visits 2, 3, 4, and 5. The entire tongue was divided into 3 parts in length and width from the root to the tip of the tongue, thus creasing 9 sections. Score 1 was given for the presence of tongue plaque in each part and score 0 was given for the absence of tongue plaque. The total tongue plaque index ranges from 0 to 9. A higher tongue plaque index indicates a greater amount of plaque. In this human test, the examiner visually checked the amount of plaque on the tongue.

The result of analysis using PP set (per protocol set) shows that, after 12 weeks of intake, the experimental group exhibited a tongue plaque index decrease of 1.53±1.28 (p<0.0001) and the control group exhibited a tongue plaque index decrease of 1.18±1.64 (p=0.0002) (FIG. 13). FIG. 13 shows the change in TPI (tongue plaque index) (PP set) upon administration of the strain combination according to the present invention.

The result of analysis using a FA set (full analysis set) shows that, after 12 weeks of intake, the experimental group exhibited a tongue plaque index decrease of 1.62±1.31 (p<0.0001) and the control group exhibited a tongue plaque index decrease of 1.10±1.61 (p=0.0001) (FIG. 14). FIG. 14 shows a change in TPI (tongue plaque index) (FA set) upon administration of the strain combination according to the present invention.

### 3) Change in GI (gingival index)

GI (gingival index) was measured on visits 2, 3, 4, and 5. Ramfjord teeth (#16, 11, 26, 36, 31, 46) were divided into buccal/lingual surfaces and evaluated as 0 to 3 scores based on the Loe & Silness scoring criteria, as shown in Table 6 below and the gingival index was calculated by dividing the total score by the number of tooth surfaces of the total teeth.

**[Table 6]**

| **Score** | Criteria |
|---|---|
| 0 | Normal gingiva |
| 1 | Gingiva with slight change in gingival color and slight swelling, but with mild inflammation not causing bleeding with light stimulation |
| 2 | Gingiva with clear symptoms of redness and swelling, and with inflammation causing bleeding even with mild stimulation |
| 3 | Gingiva with markable redness and swelling, ulcers, and severe inflammation causing natural bleeding |

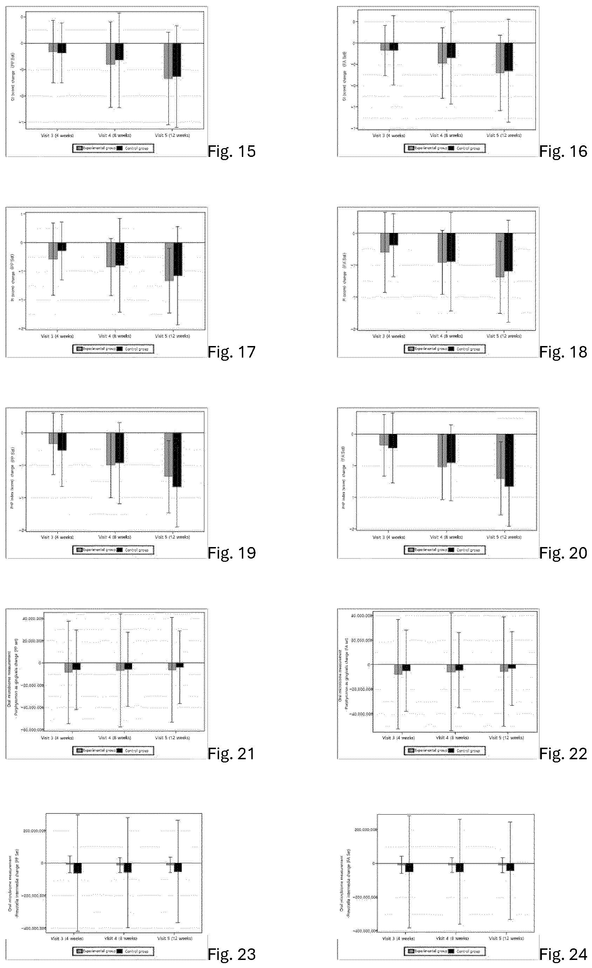

The result of analysis using PP set (per protocol set) shows that, after 8 weeks of intake, the experimental group exhibited a gingival index decrease of 0.16±0.32 (p=0.0050) and the control group exhibited a gingival index decrease of 0.13±0.36 (p=0.0424) and, after 12 weeks of intake, the experimental group exhibited a gingival index decrease of 0.27±0.35 (p<0.0001) and the control group exhibited a gingival index decrease of 0.25±0.39 (p=0.0005) (FIG. 15). FIG. 15 shows the change in GI (gingival index) (PP set) upon administration of the strain combination according to the present invention.

The result of analysis using a FA set (full analysis set) shows that, after 8 weeks of intake, the experimental group exhibited a gingival index decrease of 0.19±0.33 (p=0.0010) and the control group exhibited a gingival index decrease of 0.14±0.43 (p=0.0486) and, after 12 weeks of intake, the experimental group exhibited a gingival index decrease of 0.28±0.35 (p<0.0001) and the control group exhibited a gingival index decrease of 0.26±0.48 (p=0.0015) (FIG. 16). FIG. 16 shows a change in GI (gingival index) (FA set) upon administration of the strain combination according to the present invention.

### 4) Change in PI (plaque index)

PI (plaque index) was measured on visits 2, 3, 4, and 5. Ramfjord teeth (#16, 11, 26, 36, 31, 46) were divided into buccal/lingual surfaces and evaluated as 0 to 5 scores based on the scoring criteria of Quigley & Hein modified by Turesky et al., as shown in Table 7 below and the plaque index was calculated by dividing the total score by the number of tooth surfaces of the total teeth.

**[Table 7]**

| **Score** | Criteria |
|---|---|
| 0 | No attachment of dental plaque |
| 1 | Dotted attachment of dental plaque to the gingival margin |
| 2 | Linear attachment of dental plaque with width of less than 1 mm along the gingival margin |
| 3 | Attachment of dental plaque to 1/3 or less of cervical area |
| 4 | Attachment of dental plaque to 2/3 or less of cervical area |
| 5 | Attachment of dental plaque to 2/3 or more of cervical area |

The result of analysis using PP set (per protocol set) shows that, after 4 weeks of intake, the experimental group exhibited a plaque index decrease of 0.29±0.63 (p=0.0091) and the control group exhibited a plaque index decrease of 0.15±0.51 (p=0.1051), after 8 weeks of intake, the experimental group exhibited a plaque index decrease of 0.43±0.50 (p<0.0001) and the control group exhibited a plaque index decrease of 0.40±0.82 (p=0.0077), and, after 12 weeks of intake, the experimental group exhibited a plaque index decrease of 0.67±0.56 (p<0.0001) and the control group exhibited a plaque index decrease of 0.58±0.86 (p=0.0004) (FIG. 17). FIG. 17 shows the change in PI (plaque index) (PP set) upon administration of the strain combination according to the present invention.

The result of analysis using a FA set (full analysis set) shows that, after 4 weeks of intake, the experimental group exhibited a plaque index decrease of 0.30±0.62 (p=0.0050) and the control group exhibited a plaque index decrease of 0.19±0.49 (p=0.0212), after 8 weeks of intake, the experimental group exhibited a plaque index decrease of 0.45±0.50 (p<0.0001) and the control group exhibited a plaque index decrease of 0.45±0.77 (p=0.0007), and, after 12 weeks of intake, the experimental group exhibited a plaque index decrease of 0.69±0.56 (p<0.0001) and the control group exhibited a plaque index decrease of 0.59±0.80 (p<0.0001) (FIG. 18). FIG. 18 shows a change in PI (plaque index) (FA set) upon administration of the strain combination according to the present invention.

### 5) Change in PHP (patient hygiene performance) index

PHP (patient hygiene performance) index was measured on visits 2, 3, 4, and 5. The PHP index is a parameter that indicates plaque and oral hygiene management. The teeth were stained with a dental staining agent and the Ramfjord teeth (teeth #16, 11, 26, and 31 were measured on the buccal surface, and teeth #36 and #46 were measured on the lingual surface) were divided into five parts, namely, mesial, distal, gingival, central, and incisal parts. When plaque was attached to each part, score 1 was given, and when plaque was not attached to each part, score 0 was given. The PHP index was calculated by dividing a total of the scores for respective tooth surfaces by the number of teeth tested. It was recommended during the human test period that daily oral hygiene should be maintained, but oral hygiene treatment should not be performed (no education on brushing was provided).

The result of analysis using a PP set (per protocol set) shows that, after 8 weeks of intake, the experimental group exhibited a PHP (patient hygiene performance) index decrease of 0.50±0.50 (p<0.0001) and the control group exhibited a PHP (patient hygiene performance) index decrease of 0.47±0.62 (p=0.0001) (FIG. 19). FIG. 19 shows a change in PHP (patient hygiene performance) index (FA set) upon administration of the strain combination according to the present invention.

The result of analysis using a FA set (full analysis set) shows that, after 8 weeks of intake, the experimental group exhibited a PHP (patient hygiene performance) index decrease of 0.52±0.51 (p<0.0001) and the control group exhibited a PHP (patient hygiene performance) index decrease of 0.45±0.60 (p<0.0001) (FIG. 20). FIG. 20 shows a change in PHP (patient hygiene performance) index (FA set) upon administration of the strain combination according to the present invention.

### 6) Oral microbiome measurement

Oral microbiome was measured on visits 1, 3, 4, and 5. In this human test, the bacteria causing bad breath (*Porphyromonas gingivalis, Prevotella intermedia, Fusobacterium nucleatum, Streptococcus mutans*) were analyzed by qPCR. For oral microbiome measurement, saliva was collected in a tube containing a preservative solution, stored in a refrigerator (2-8°C) or at room temperature, and analyzed by an external analysis agency. After the analysis was completed, the samples were discarded.

### A. Change in P. gingivalis

The result of analysis using a PP set (per protocol set) shows that, after 12 weeks of intake, the experimental group exhibited a *P*. *gingivalis* decrease of 6,303,000.00147,074,074.92 CFU/g (p=0.4338), and the control group exhibited a *P*. *gingivalis* decrease of 3,942,212.12±32,735,987.56 CFU/g (p=0.4941) (FIG. 21). FIG. 21 shows a change in *P. gingivalis* upon administration of the strain combination according to the present invention.

The result of analysis using a FA set (full analysis set) shows that, after 12 weeks of intake, the experimental group exhibited a P. gingivalis decrease of 11,284,000.00146,191,102.86 CFU/g (p=0.1575) and the control group exhibited a P. gingivalis decrease of 52,499,090.91±313,804,155.21 CFU/g (FIG. 22). FIG. 22 shows a change in *P. gingivalis* (FA set) upon administration of the strain combination according to the present invention.

### B. Change in P. intermedia

The result of analysis using a PP set (per protocol set) shows that, after 12 weeks of intake, the experimental group exhibited a *P*. *intermedia* decrease of 11,284,000.00146,191,102.86 CFU/g (p=0.1575) and the control group exhibited a *P*. *intermedia* decrease of 52,499,090.911313,804,155.21 CFU/g (p=0.3437) (FIG. 23). FIG. 23 shows a change in *P*. *intermedia* upon administration of the strain combination according to the present invention.

The result of analysis using a FA set (full analysis set) shows that, after 12 weeks of intake, the experimental group exhibited a P. intermedia decrease of 10,558,974.36±43,802,128.15 CFU/g (p=0.1405) and the control group exhibited a P. intermedia decrease of 44,065,384.621288,684,314.48 CFU/g (p=0.3465) (FIG. 24). FIG. 24 shows a change in *P*. *intermedia* (FA set) upon administration of the strain combination according to the present invention.

### C. Change in F. nucleatum

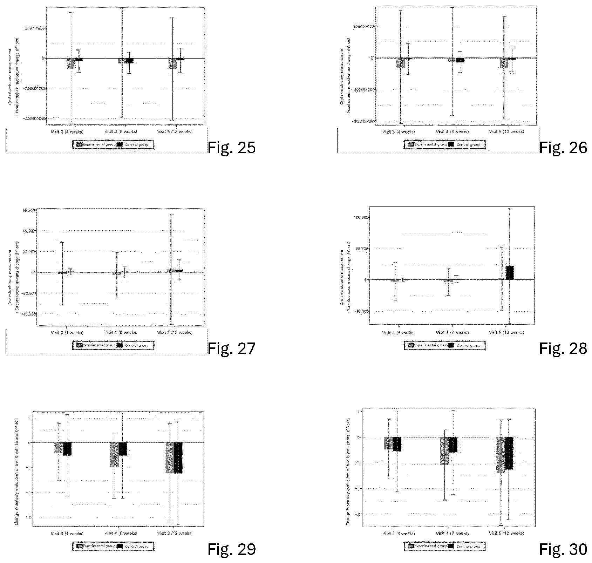

The result of analysis using a PP set (per protocol set) shows that, after 12 weeks of intake, the experimental group exhibited a *F. nucleatum* decrease of 688,485,714.29±3,426,961,202.10 CFU/g (p=0.2429) and the control group exhibited a F. nucleatum decrease of 144,757,575.76±828,219,325.96 CFU/g (p=0.3229) (FIG. 25). FIG. 25 shows a change in *F. nucleatum* upon administration of the strain combination according to the present invention.

The result of analysis using a FA set (full analysis set) shows that, after 12 weeks of intake, the experimental group exhibited a *F. nucleatum* decrease of 619,051,282.05±3,248,312,549.36 CFU/g (p=0.2414) and the control group exhibited a *F. nucleatum* decrease of 115,128,205.131779,278,383.46 CFU/g (p=0.3620) (FIG. 26). FIG. 26 shows a change in *F. nucleatum* (FA set) upon administration of the strain combination according to the present invention.

### D. Change in S. mutans

The result of analysis using a PP set (per protocol set) shows that, after 8 weeks of intake, the experimental group exhibited a *S. mutans* decrease of 2,623.53122,133.36 CFU/g (p=0.4943) and the control group exhibited a 484.85±5,093.63 CFU/g increase (p=0.5883) (FIG. 27). FIG. 27 shows a change in *S. mutans* upon administration of the strain combination according to the present invention.

The result of analysis using a FA set (full analysis set) shows that, after 8 weeks of intake, the experimental group exhibited a *S*. *mutans* decrease of 3,400.00±21,785.79 CFU/g (p=0.3423) and the control group exhibited a *S. mutans* decrease of 923.08±5,630.67 CFU/g (p=0.3124) (FIG. 28). FIG. 28 shows a change in *S. mutans* (FA set) upon administration of the strain combination according to the present invention.

### 7) Sensory evaluation of bad breath

A bad breath sensory evaluation was conducted on visits 2, 3, 4, and 5. The subjects of the human test were asked to keep their mouths closed for 2 minutes and then exhale gently. At this time, bad breath was evaluated by smell at a distance of about 10 cm from the front of the subject, on a scale of 0 to 5, as shown in Table 8 below. They were advised not to eat certain foods (garlic, onions, etc.) that could affect bad breath the day before the visit.

**[Table 8]**

| **Score** | Criteria |
|---|---|
| 0 | No bad breath |
| 1 | Barely perceptible bad breath |
| 2 | Weak bad breath |
| 3 | Medium bad breath |
| 4 | Strong bad breath |
| 5 | Very strong bad breath |

The result of analysis using a PP set (per protocol set) shows that, after 8 weeks of intake, the experimental group exhibited a bad breath decrease of 0.47±0.65 (p=0.0001) and the control group exhibited a bad breath decrease of 0.26±0.86 (p=0.0831) (FIG. 29). FIG. 29 shows a change in bad breath upon administration of the strain combination according to the present invention.

The result of analysis using a FA set (full analysis set) shows that, after 8 weeks of intake, the experimental group exhibited a bad breath decrease of 0.54±0.68 (p<0.0001) and the control group exhibited a bad breath decrease of 0.30±0.82 (p=0.0265) (FIG. 30). FIG. 30 shows a change in bad breath (FA set) upon administration of the strain combination according to the present invention.

### 3. Stability Evaluation

### 1) Stability Variables

### A. Abnormal cases

All abnormal cases that occurred after consumption of the food for human tests were tabulated and evaluated based on a calculated occurrence rate. The ratio of subjects in the human test who experienced abnormal cases in each group was calculated and compared and analyzed using the chi-square test or Fisher's exact test.

### B. Vital Signs (blood pressure and pulse), physical measurements (weight)

The comparison in the changes of vital signs (blood pressure, pulse) and physical measurements (weight) in the groups before and after intake was analyzed using the Paired t-test, and the degree of change between the experimental group and the control group was evaluated for statistically significant differences by performing a two-sample t-test or Wilcoxon Rank Sum test depending on whether or not normality was satisfied.

### D. Clinical pathology test (hematology/blood chemistry test, urine test)

The comparison in changes of hematology and blood test results in the groups before and after intake was analyzed using a paired t-test and the degree of change between the experimental group and the control group was evaluated for statistically significant differences by performing the two-sample t-test or Wilcoxon rank sum test depending on whether or not normality was satisfied. The urine test was performed by performing a MCNemar test on normal and abnormal groups and comparing the difference between the groups.

### 2) Results of stability evaluation

The stability evaluation was conducted on subjects of human tests who consumed the food for human tests at least once after being randomly assigned to the human test, and a total of 80 subjects (40 in the experimental group and 40 in the control group) of human test subjects were included in the analysis subjects.

No abnormal cases occurred in either the experimental group or the control group, and there were no statistically significant differences between the intake groups. No serious abnormal cases occurred and there were no dropouts due to abnormal cases.

In this human test, clinical pathology tests for stability evaluation were conducted at visits 1 and 5. The test items were divided into hematological tests, blood chemistry tests, and urine tests and evaluated.

### A. Blood chemistry test

In the blood chemistry test, after 12 weeks of intake, the experimental group exhibited a glucose decrease of 4.16±7.07 mg/dL (p=0.0009), while the control group exhibited a glucose increase of 1.82±12.45 mg/dL (p=0.3744), which indicates a statistically significant difference between the intake groups (p=0.0094).

In the blood chemistry test, after 12 weeks of intake, the experimental group exhibited a P increase of 0.18±0.54 mg/dL (p=0.0447), while the control group exhibited a P decrease of 0.08±0.60 mg/dL (p=0.4041), which indicates a statistically significant difference between the intake groups (p=0.0471).

In addition, there were no statistically significant differences in blood chemistry test items in either the experimental group or the control group after 12 weeks of intake.

That is, there were no statistically significant differences between the intake groups in all items of hematological and urine tests after 12 weeks of intake.

### B. Vital signs (pulse, blood pressure) and physical measurements (weight)

The experimental group and the control group after 12 weeks of intake had normal vital signs (pulse, blood pressure) and physical measurements (weight) and there were no statistically significant differences therebetween.

Therefore, the tablets of both the experimental group and the control group are recognized as stable.

### [Accession number]

Name of deposit organization: the Korea Center for Microorganism Conservation (KCCM)
Accession number: KCCM13365P
Acceptance date: 20230630

As is apparent from the above description, the strain combination according to the present invention is highly effective in inhibiting oral harmful bacteria and thus in ameliorating, preventing, and treating oral diseases including bad breath, tooth decay, and periodontal disease.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. A pharmaceutical composition for preventing or treating an oral disease comprising a *Lactobacillus gasseri* HHuMIN D strain (KCCM 12724P) and a *Lacticaseibacillus paracasei* OK strain (KCCM 13365P).

2. The pharmaceutical composition according to claim 1, wherein the pharmaceutical composition contains a combination of any one selected from live cells and dead cells of a *Lactobacillus gasseri* HHuMIN D strain (KCCM 12724P), and a culture solution thereof, and any one selected from live cells and dead cells of a *Lacticaseibacillus paracasei* OK strain (KCCM 13365P), and a culture solution thereof.

3. The pharmaceutical composition according to claim 1, wherein the oral disease is selected from bad breath, caries and periodontal disease.

4. The pharmaceutical composition according to claim 3, wherein the bad breath is caused by *Fusobacterium nucleatum.*

5. The pharmaceutical composition according to claim 3, wherein the caries is caused by *Streptococcus mutans.*

6. The pharmaceutical composition according to claim 3, wherein the periodontal disease is caused by any one selected from the group consisting of *Fusobacterium nucleatum, Porphyromonas gingivalis,* and *Prevotella intermedia.*

7. A food composition for ameliorating an oral disease comprising a *Lactobacillus gasseri* HHuMIN D strain (KCCM 12724P) and a *Lacticaseibacillus paracasei* OK strain (KCCM 13365P).

8. The food composition according to claim 7, wherein the food composition contains a combination of any one selected from live cells and dead cells of a *Lactobacillus gasseri* HHuMIN D strain (KCCM 12724P), and a culture solution thereof, and any one selected from live cells and dead cells of a *Lacticaseibacillus paracasei* OK strain (KCCM 13365P), and a culture solution thereof.

9. A composition for suppressing bad breath comprising a *Lactobacillus gasseri* HHuMIN D strain (KCCM 12724P) and a *Lacticaseibacillus paracasei* OK strain (KCCM 13365P).

10. The composition according to claim 9, wherein the composition contains a combination of any one selected from live cells and dead cells of a *Lactobacillus gasseri* HHuMIN D strain (KCCM 12724P), and a culture solution thereof, and any one selected from live cells and dead cells of a *Lacticaseibacillus paracasei* OK strain (KCCM 13365P), and a culture solution thereof.
